# EUROPEAN PATENT APPLICATION

(11) **EP 4 631 560 A1**
(43) Date of publication of application: **15.10.2025**
(21) Application number: 24184903.3
(22) Date of filing: 27.06.2024
(51) Int. Cl.: A61M 25/00

(54) **GUIDE EXTENSION CATHETER**

(30) Priority: 10.04.2024 CN 202410430370
(71) Applicant: Orbusneich Medical (Shenzhen) Co., Ltd., 518038 Shenzhen (CN)
(72) Inventor: ZHANG, Lu, Shenzhen, 518038 (CN); GUO, Lixia, Shenzhen, 518038 (CN)
(74) Representative: Cabinet Chaillot

(57) **Abstract**

The present application discloses a guide extension catheter, comprising: a guide tube, which comprises a proximal port and a distal port and comprises an inner layer, an intermediate layer, and an outer layer; a guide shaft, which is disposed on the side of proximal port and configured to guide the guide tube into a target position; a transitional connection segment, which is configured to connect the guide tube and the guide shaft, wherein the transitional connection segment is of a tubular structure that axially extends from the proximal port and comprises an inclined opening, and comprises an inner-layer extension segment, an outer-layer extension segment, and a rib component; the rib component is sized and shaped to adapt to the transitional connection segment; the rib component and the intermediate layer are coaxial with but not connected to each other, and are spaced apart by a first spacing of 0.1 mm to 10 mm; the rib component is spaced apart from the guide shaft by a third spacing of 0.1 mm to 2 mm; and the intermediate layer, the rib component, the inner layer and the inner-layer extension segment are wrapped by the outer layer and the outer-layer extension segment and are then hot-melt welded together. The guide extension catheter has both support strength and flexibility, and achieves comprehensive and significant improvements in terms of the passability, pushability, effective cavity channel retention capability, and reliability.

## Description

### TECHNICAL FIELD

The present application relates to the field of medical products, and in particular to a guide extension catheter.

### BACKGROUND ART

A guide extension catheter, which is used cooperatively with a guide tube or a sheath during a vascular interventional operation, enters the coronary artery to assist in the placement of an interventional treatment instrument. In the existing guide extension catheter technology, a transitional connection segment is designed generally into two types, including a first type of plastic skirt transition and a second type of metal skirt transition. The plastic skirt transition has structural features that a metal reinforcement layer of a guide tube is directly connected to a metal guide shaft to ensure the tensile strength, a plastic layer of the guide tube extends to the guide shaft and is cut in a transition region to form an arc-shaped or step-shaped plastic inclined cut (or skirt) that also serves as an entrance to a lumen of the guide tube, and the transition formed by the arc-shaped or step-shaped plastic inclined cut has the advantage that the main part of the transitional connection segment is made of plastic and thus has good flexibility, and has the disadvantage that the plastic skirt has poor mechanical transmission and mechanical transition due to the poor stiffness of plastic, which causes large stress concentration, easy deformation resulting in loss of entrance to the lumen and irreversible deformation, and poor tensile strength, so that in actual use, the guide extension catheter cannot be placed at a designated position due to insufficient push force transmission, or the guide extension catheter may fail or break due to the loss of entrance to the lumen caused by the deformation of the transitional connection segment. The metal skirt transition has structural features that the end of a guide shaft is connected (generally welded or riveted) to a metal ring that is then connected to a plastic layer of a guide tube in a covered and inlaid manner to ensure the tensile strength, the metal ring generally extends from the end of the plastic layer of the guide tube to the guide shaft and is cut in a transition region to form an arc-shaped or step-shaped metal skirt that also serves as an entrance to a lumen of the guide tube, and the transition formed by the arc-shape or step-shaped metal skirt has the advantage that the metal structure well support the entrance to the lumen and is thus not easy to collapse, resulting in good mechanical transmission and good tensile strength, and has the disadvantage that the transitional connection segment has poor performance to pass a bend and poor mechanical transition due to being too stiff, which leads to large stress concentration at the junction between the end of the metal ring and the guide tube, resulting in bending of the tube body at the junction and then the loss of the lumen, so that in actual use, the guide extension catheter cannot reach a designated position to play its role due to the poor performance to pass a bend, or the patient's blood vessel is damaged due to excessive resistance during the advancement.

### SUMMARY OF THE INVENTION

In one aspect, the present application provides a guide extension catheter, including:
a guide tube, which includes a proximal port and a distal port and includes an inner layer, an intermediate layer, and an outer layer; a guide shaft, which is disposed on the side of proximal port and configured to guide the guide tube into a target position; a transitional connection segment, which is configured to connect the guide tube and the guide shaft, the transitional connection segment being of a tubular structure that axially extends from the proximal port and includes an inclined opening, wherein the transitional connection segment includes an inner-layer extension segment, an outer-layer extension segment, and a rib component; the rib component is sized and shaped to adapt to the transitional connection segment; and the inner-layer extension segment is a part of the inner layer, and the outer-layer extension segment is a part of the outer layer; wherein the rib component and the intermediate layer are coaxial with but not connected to each other, and are spaced apart by a first spacing of 0.1 mm to 10 mm; and the rib component and the guide shaft are spaced apart by a distance but not connected to each other, and are spaced apart by a third spacing of 0.1 mm to 2 mm; and wherein the intermediate layer, the rib component, the inner layer and the inner-layer extension segment are wrapped by the outer layer and the outer-layer extension segment and are then hot-melt welded together.

At least some embodiments have the advantages that the transitional connection segment of the guide extension catheter includes a rib component that is not connected to any adjacent metal components, so that the transitional connection segment has superior flexibility, strong cavity supporting capability, excellent mechanical transmission and transition, and super-strong memory restoration capability, and thus compared with the prior art, the guide extension catheter achieves comprehensive and significant improvements in terms of key properties including the passability, pushability, effective cavity channel retention capability, and reliability.

### BRIEF DESCRIPTION OF THE DRAWINGS

The features and advantages of the present application may be further understood with reference to the remaining parts of this description and the accompanying drawings. Identical assemblies in these accompanying drawings are numbered with the same reference numerals. In some cases, a sub-marker is shown after a reference numeral and hyphen to represent one of many similar assemblies. Any one mentioned reference numeral with an existing sub-marker not specifically identified refers to all such similar assemblies.
FIG. 1 is a schematic diagram of a usage scenario of a guide extension catheter according to an embodiment of the present application.
FIG. 2 is a schematic structural diagram of a guide extension catheter according to an embodiment of the present application.
FIG. 3 is a cross-sectional view of the guide extension catheter according to the embodiment depicted in FIG. 2 along line 3-3.
FIG. 4A is a partial schematic front view of the structure shown in FIG. 3.
FIG. 4B is a partial schematic top view of the structure shown in FIG. 3.
FIG. 4C is a partial schematic side view of the structure shown in FIG. 3.
FIG. 5A is a front view of a rib component structure according to one of the embodiments of the present application.
FIG. 5B is a top view of the rib component structure according to the embodiment of the present application shown in FIG. 5A.
FIG. 5C is a left view of the rib component structure according to the embodiment of the present application shown in FIG. 5A.
FIG. 6A is a front view of the rib component structure according to another embodiment of the present application.
FIG. 6B is a side view of the rib component structure according to the embodiment of the present application shown in FIG. 6A.
FIG. 7A is a front view of the rib component structure according to still another embodiment of the present application.
FIG. 7B is a side view of the rib component structure according to the embodiment of the present application shown in FIG. 7A.
FIG. 8A is a front view of a rib component structure according to yet another embodiment of the present application.
FIG. 8B is side view of the rib component structure according to the embodiment of the present application shown in FIG. 8A.

### DETAILED DESCRIPTION OF EMBODIMENTS

The embodiments are described in greater detail below with reference to the following examples, which are provided herein by way of illustration only and are not intended to be limiting.

The present application has many variations which can be expected by those skilled in the art, and the use effect of the present application can be achieved.

In the present application, space or position defining terms, such as inner, outer, upper, lower, left, right, top, bottom, front side and back side, are defined with reference to a placement position of a guide extension catheter in a use state.

In the present application, the term "including" means including, but not limited to, the following elements, that is, not excluding other elements.

In the present application, when the guide extension catheter is in use, in two ends of the guide extension catheter itself or any part of the guide extension catheter, the end relatively close to an operator's hand is a "proximal end", and correspondingly, the end relatively away from the operator's hand is a "distal end".

In the present application, the term "axial direction" refers to a direction in which the central axis of the guide extension catheter or an extension line of the central axis is located.

In the present application, the term "circumferential direction" refers to a direction in which a circumferential wall of the guide extension catheter is located.

In the present application, the term "oblique direction" refers to a direction of a line intersecting the central axis of the guide tube at an acute angle.

In the present application, the term "connected" refers to a physical rigid connection between two particular parts, including but not limited to physical connection methods such as welding, soldering, and riveting. The term "not connected" means that the two particular parts are not directly connected physically and rigidly.

In the present application, the terms "about" and "approximately" refer to accuracy intervals that would be understood by those skilled in the art so as to still ensure the technical effects of the features discussed. The terms generally denote a deviation of 10%, preferably 5%, from an indicated value.

A transitional connection segment of the guide extension catheter located between a guide tube and a guide shaft is the key part of the whole guide extension catheter, and the quality of its connection performance and transition performance determines whether the guide extension catheter can play its role in actual use. During the insertion and pushing of the guide extension catheter into a human body, there are usually tortuous passages or narrow blood vessels. In this case, since the guide tube has a larger cross section and is thus easy to be blocked, and the guide shaft has a smaller cross section, the ratio of cross-sectional area thereof is generally large (for example, 80 : 1), and they are structurally different in hardness, so that during the actual advancement of the guide extension catheter, a large stress concentration usually occurs at the transitional connection segment, which makes it easy to deform and bend at the transition portion. As a result, the coaxiality of the mechanical transmission of the guide extension catheter is destroyed, such that the push force applied from an operating end of the guide shaft outside of the body cannot be effectively transmitted to a guide tube body via the transitional connection segment, and thus the guide extension catheter cannot continue to advance and reach the designated position to play its role. The deformation of the transitional connection segment during operation may also directly lead to deformation at the entrance to the lumen of the guide tube, and the deformation occurring at the entrance to the lumen can easily lead to the loss of effective lumen of the guide extension catheter, such that an originally matched interventional treatment instrument will be blocked at the entrance to the guide tube and cannot enter the lumen, making the guide extension catheter ineffective. During the withdrawal of the guide extension catheter, the guide tube may be stuck due to deformation or channel collapse, resulting in higher resistance to the withdrawal of the guide tube than normal. In this case, the overall tensile strength of the guide extension catheter is put to the proof, and due to the aforementioned structural reasons, the transitional connection segment is usually a weak part that is more likely damaged first when being pulled, which causes the product to break and endanger the patient's life.

The present application provides a guide extension catheter having a free rib component, and the transitional connection segment thereof includes the free rib component that is not connected to any metal component, such that the transitional connection segment has superior flexibility, strong lumen supporting capability, excellent mechanical transmission and transition, and super-strong memory restoration capability, and the guide extension catheter achieves comprehensive and significant improvements on several key properties, such as the passability, pushability, effective cavity channel retention capability, and reliability. Further, the guide extension catheter can fully and reliably play its role in a surgical operation, which is conducive to shortening the surgical operation time and improving the success rate of the operation. The specific technical solution thereof is as follows.

In one aspect, there is provided a guide extension catheter, including:
a guide tube, which includes a proximal port and a distal port and includes an inner layer, an intermediate layer, and an outer layer; a guide shaft, which is disposed on the side of proximal port and configured to guide the guide tube into a target position; a transitional connection segment, which is configured to connect the guide tube and the guide shaft, the transitional connection segment being of a tubular structure that axially extends from the proximal port and includes an inclined opening, wherein the transitional connection segment includes an inner-layer extension segment, an outer-layer extension segment, and a rib component; the rib component is sized and shaped to adapt to the transitional connection segment; the inner-layer extension segment is a part of the inner layer, and the outer-layer extension segment is a part of the outer layer; wherein the rib component and the intermediate layer are coaxial with but not connected to each other, and are spaced apart by a first spacing of 0.1 mm to 10 mm; and the rib component and the guide shaft are spaced apart by a distance but not connected to each other, and are spaced apart by a third spacing of 0.1 mm to 2 mm; and wherein the intermediate layer, the rib component, the inner layer and the inner-layer extension segment are wrapped by the outer layer and the outer-layer extension segment and are then hot-melt welded together. The rib component provides rigid support for the transitional connection segment, and the rib component is not connected to the intermediate layer of the guide tube, which enhances the flexibility of the transitional connection segment and enables the guide extension catheter to achieve the overall balance between the stiffness and flexibility.

In at least one embodiment, the rib component includes a circumferential rib and an oblique rib, the oblique rib having a profile corresponding to the shape of the inclined opening; and the circumferential rib and the oblique rib form an overlapping connection segment at the proximal port, such that the circumferential rib and the oblique rib form an integral structure as a whole.

In at least one embodiment, the circumferential rib is C-shaped and includes two arc-shaped ribs circumferentially extending from two ends of the overlapping connection segment.

In at least one embodiment, the circumferential rib is a circumferential closed ring that passes the overlapping connection segment.

In at least one embodiment, the oblique rib is C-shaped and includes a left rib and a right rib which are sized and positioned corresponding to the inclined opening, and multiple sets of auxiliary circumferential ribs are distributed within a length range of the left rib and the right rib to support the circumferential stiffness of the transitional connection segment.

In at least one embodiment, tail portions of the auxiliary circumferential ribs do not overlap each other, and are spaced apart by a distance.

In at least one embodiment, the circumferential rib or the auxiliary circumferential rib is circumferentially S-shaped.

In at least one embodiment, the circumferential rib or the auxiliary circumferential rib includes a basic segment and a reinforcement segment, the reinforcement segment having a larger width or surface area than the basic segment.

In at least one embodiment, the reinforcement segment includes a branch or an eyelet.

In at least one embodiment, the rib component is made of a nickel-titanium alloy, a cobalt alloy, a titanium alloy, a platinum-iridium alloy, stainless steel, or a carbon fiber material. In at least one embodiment, the rib component is made of a nickel-titanium alloy, a cobalt alloy, a titanium alloy, a platinum-iridium alloy, stainless steel, or a carbon fiber material, and forms a round wire or a square bar.

In at least one embodiment, the outer layer is made of nylon, polyurethane or a thermoplastic elastomer, and the inner layer is made of a polymer; and the intermediate layer is a metal mesh layer.

In another aspect, the present application provides a guide extension catheter, including:
a guide tube, which includes a proximal port and a distal port and includes an inner layer, an intermediate layer, and an outer layer, the inner layer and the outer layer being made of plastic; a guide shaft, which is disposed on the side of proximal port and configured to guide the guide tube into a target position; a transitional connection segment, which is configured to connect the guide tube and the guide shaft, the transitional connection segment being of a tubular structure that axially extends from the proximal port and includes an inclined opening, wherein the transitional connection segment includes an inner-layer extension segment, an outer-layer extension segment, and a rib component; the rib component includes a C-shaped circumferential rib and a C-shaped oblique rib, the oblique rib having a profile corresponding to the shape of the inclined opening; the circumferential rib and the oblique rib form an overlapping connection segment at the proximal port, such that the circumferential rib and the oblique rib form an integral structure as a whole; and the oblique rib includes a left rib and a right rib which are sized and positioned corresponding to the inclined opening, and multiple sets of auxiliary circumferential ribs are distributed within the length range of the left rib and the right rib to support the circumferential stiffness of the transitional connection segment; wherein the rib component and the intermediate layer are coaxial with but not connected to each other, and are spaced apart by a first spacing of 0.1 mm to 10 mm; and the rib component and the guide shaft are spaced apart by a distance but not connected to each other, and are spaced apart by a third spacing of 0.1 mm to 2 mm; and wherein the intermediate layer, the rib component, the inner layer and the inner-layer extension segment are wrapped by the outer layer and the outer-layer extension segment and are then hot-melt welded together.

The aspects of the embodiments of the present application will be described in detail below with reference to the drawings.

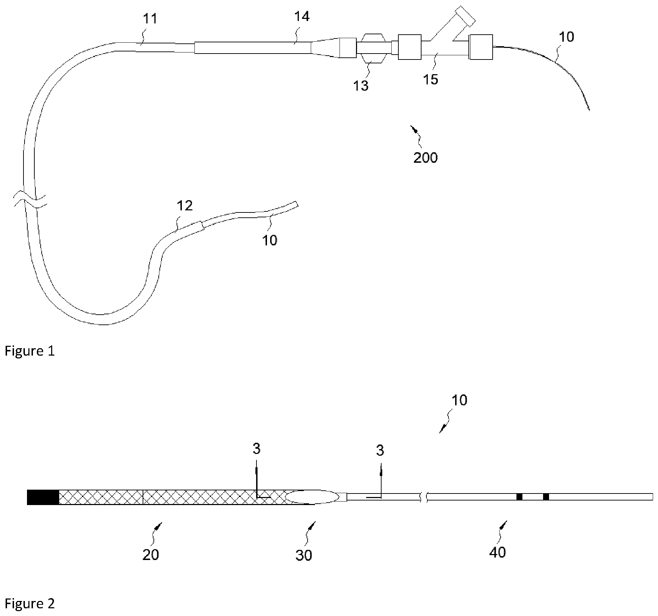

As shown in FIG. 1, it is a schematic diagram of the guide extension catheter 10 used cooperatively with a matching instrument 200. The matching instrument 200 includes a primary interventional channel formed by the guide tube 11 and a guide sheath 14 jointly. A distal end 12 of the guide tube enters a blood vessel, a proximal end 13 of the guide tube is connected to a Y-type hemostasis valve 15, and the guide extension catheter 10 enters the primary interventional channel via the Y-type hemostasis valve 15, advances along the lumen of the guide tube 11, and finally extends out of the distal end 12 of the guide tube and is then placed into a more distant blood vessel (such as the coronary artery).

FIG. 2 is a diagram of external view of the guide extension catheter 10 described in one of the embodiments of the present application. As shown in FIG. 2, the guide extension catheter 10 includes three parts, namely, a guide tube 20, a transitional connection segment 30, and a guide shaft 40. The guide tube is also referred to as a distal tube, the guide shaft is also referred to as a proximal shaft, and the transitional connection segment may also be referred to as a transition portion. The guide tube 20 is located at the distal end of the guide extension catheter 10, the guide shaft 40 is located at the proximal end of the guide extension catheter 10, and the transitional connection segment 30 is configured to achieve the connection between the guide tube 20 and the guide shaft 40.

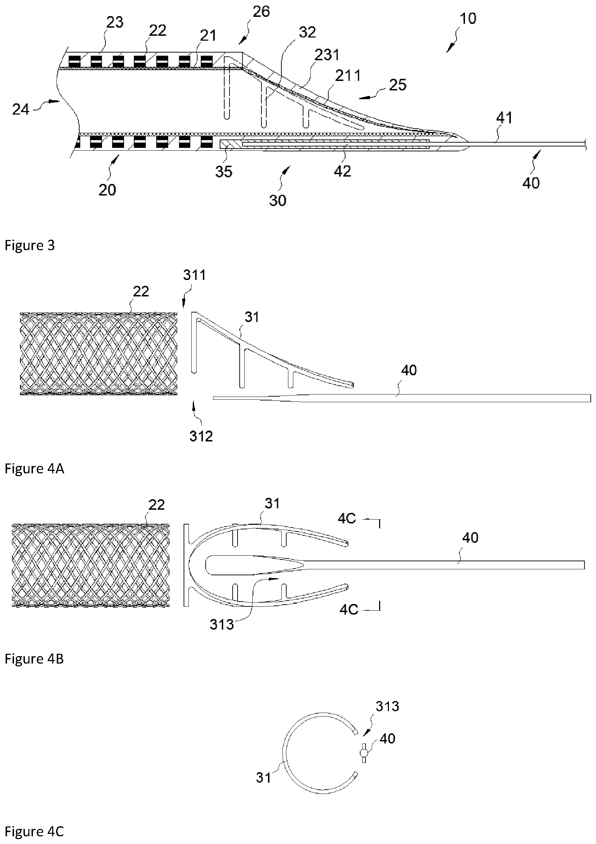

FIG. 3 is an axial cross-sectional view of the guide extension catheter described in one of the embodiments of the present application. As shown in FIG. 3, the guide tube 20 is of a tubular structure and serves as a channel for an interventional medical device, and the guide tube 20 structurally includes, from inside to outside from the lumen, an inner layer 21, an intermediate layer 22, and an outer layer 23. The inner layer 21, the intermediate layer 22 and the outer layer 23 form a tubular structure including a lumen 24. The inner layer 21 may be made of PTFE or other plastics, the intermediate layer 22 may be made of a stainless steel woven mesh or other metal woven meshes, and the outer layer 23 may be made of a thermoplastic elastomer, a polyurethane elastomer or other plastics. The guide shaft 40 is an elongated solid rod, and includes a body portion 41 and a distal portion 42. The body portion 41 may be a cylindrical stainless steel rod or other metal rod, the distal portion 42 is an extension of the body portion 41 formed by stamp-flattening, laser cutting and flattening, grinding and thinning, or other treatment methods, and the distal portion 42 may have a length of 1 mm to 50 mm. As can be seen from FIG. 3, the transitional connection segment 30 is a part axially extending from a proximal port 26 of the guide tube 20, connects the guide tube 20 and the guide shaft 40 together, and has an inclined opening 25, the inclined opening 25 being in communication with the lumen 24 of the guide tube 20. The transitional connection segment 30 includes an inner-layer extension segment 211, an outer-layer extension segment 231, and a rib component 31. The rib component 31 is wrapped between the inner-layer extension segment 211 and the outer-layer extension segment 231, the rib component 31 and a proximal end of the intermediate layer 22 are coaxial with but not connected to each other, and may be spaced apart by a distance of 0.1 mm to 10 mm, and the rib component 31 and the guide shaft 40 are not connected to each other and are spaced apart by a distance of 0.1 mm to 2 mm, which has a function to enhance the structural flexibility and avoid the increase in thickness caused by stacking to reduce the overall structural thickness. The rib component 31 may be made of a nickel-titanium alloy, a cobalt alloy, a titanium alloy, a platinum-iridium alloy, stainless steel, a carbon fiber, or other metals, and may also be a round wire or a square bar or in any bar-shaped form. The rib component may be integrally formed as a whole, or may be formed as a whole by means of segmented welding, etc. The periphery of the distal portion 42 of the guide shaft 40 is coated with a bridging wedge sleeve 35 by means of hot-melt coating or stamping, welding, soldering, riveting, etc., and the bridging wedge sleeve 35 may be made of plastic such as nylon, or metal. The bridging wedge sleeve 35 is coated and integrally embedded between the outer layer 23 and the guide shaft 40 to form a bridging tenon-mortise structure, which greatly enhances the structural firmness and tensile strength while maintaining the structural flexibility. The rib component 31 is also coated and integrally embedded between the outer layer 23 and the inner layer 21, and after the redundant tube body portion without the intermediate layer 22 and the rib component 31 is removed at the proximal end of the guide tube 20, an arc-shaped bevel, that is, an inclined opening 25, is finally formed, and the inclined opening 25 is also an entrance for other interventional instruments to enter the guide extension catheter 10. At this point, after being assembled, the above components may be hot-melt coated by the outer layer 23 of the guide tube 20 as a whole, which forms a main structure of the guide extension catheter 10.

The proximal end of the guide tube 20, the rib component 31, the bridging wedge sleeve 35, and a distal end of the guide shaft 40 jointly form the transitional connection segment 30 of the guide extension catheter, and the presence of the rib component 31 greatly alleviates the large stress concentration at both ends of the transitional connection segment 30 due to the large cross-sectional area difference (for example, 80 : 1) and the structural difference of hardness, thereby significantly improving the mechanical transmission and transition of the transitional connection segment 30. Also, the rib component 31 made of a nickel-titanium alloy, a cobalt alloy, a titanium alloy, a platinum-iridium alloy, stainless steel, or carbon fiber can significantly improve the cavity channel supporting capability of the inclined opening 25 and the memory restoration capability after deformation under force, the structural design of the rib component 31 considers the flexibility that adapts to deformation at any angle, and also helps to improve the capacity of the transitional connection segment 20 to pass a bend, thereby significantly improving the overall key properties of the guide extension catheter 10, including the passability, pushability, effective cavity channel retention capability, and reliability.

FIGS. 4A to 4C show simple partial schematic diagrams of the structure shown in FIG. 3, and FIG. 4C is a cross-sectional view taken along line 4C-4C in FIG. 4B. The figures illustrate the relative positions of the main components of a framework structure of the guide extension tube, mainly including the intermediate layer 22, the rib component 31, and the guide shaft 40. The rib component 31 is coaxial with the proximal end of the intermediate layer 22 and located above the guide shaft 40, the rib component 31 is coaxial with but not connected to the proximal end of the intermediate layer 22, the rib component 31 and the intermediate layer 22 are spaced apart by a first spacing 311, and the distance of the first spacing 311 may be 0.1 mm to 10 mm. The proximal end of the intermediate layer 22 and the distal end of the guide shaft 40 are aligned with but not connected to each other, and are spaced apart by a second spacing 312, and the distance of the second spacing 312 may be 0.1 mm to 10 mm. The relative position relationship between the rib component 31 and the guide shaft 40 is as shown in FIG. 4, the rib component 31 and the guide shaft 40 are distributed in the circumferential direction of the transitional connection segment, and are spaced apart by a third spacing 313, and the distance of the third spacing 313 may be 0.1 mm to 2 mm. The spacing design described above enhances the structural flexibility and avoids the increase in thickness caused by stacking to reduce the overall structural thickness, so that the rib component provides sufficient rigid support for the transitional connection segment while avoiding stacking, thereby significantly improving the mechanical transmission and transition of the transitional connection segment 30.

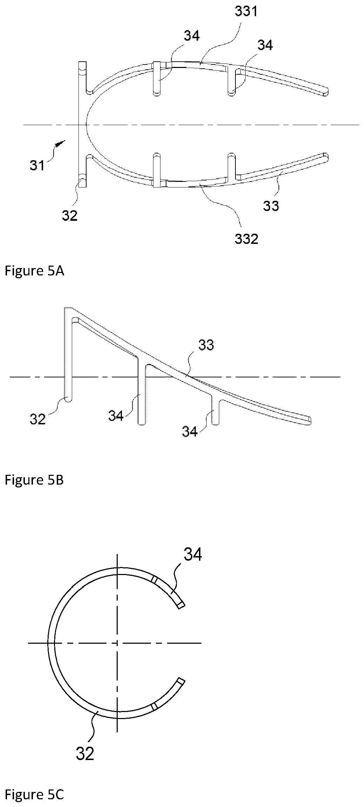

FIGS. 5A to 5C are respectively a front view, a top view and a left view of the rib component structure according to one of the embodiments of the present application. As shown in FIG. 5A, the rib component 31 includes a plurality of ribs, including a circumferential rib 32, an oblique rib 33, and auxiliary circumferential ribs 34. As shown in FIG. 5C, the circumferential rib 32 is C-shaped in the circumferential direction, and is distributed perpendicular to the axial direction of the guide extension catheter. The circumferential rib 32 and the oblique rib 33 form an overlapping connection segment at the proximal port 26 of the guide tube, the profile of the oblique rib 33 corresponds to the shape of the inclined opening 25 and includes a left rib 331 and a right rib 332, and the left rib 331 and the right rib 332 are sized and positioned corresponding to the inclined opening and are configured to support the circumferential stiffness of the transitional connection segment. The oblique rib 33 intersects the axial direction of the guide extension catheter at a certain angle and/or radian. Multiple pairs of auxiliary circumferential ribs 34 are distributed within the length range of the left rib 331 and the right rib 332, the auxiliary circumferential ribs 34 are C-shaped in the circumferential direction, and may be two or more pairs. The rib component 31 may be machined by means of laser cutting, wire cutting, welding, bonding, or other methods. In some embodiments, the circumferential rib 32 of the rib component 31, and the auxiliary circumferential ribs 34 may be O-shaped in the circumferential direction, that is, connected end-to-end to form a closed ring. In some embodiments, the oblique rib 33 may also be O-shaped.

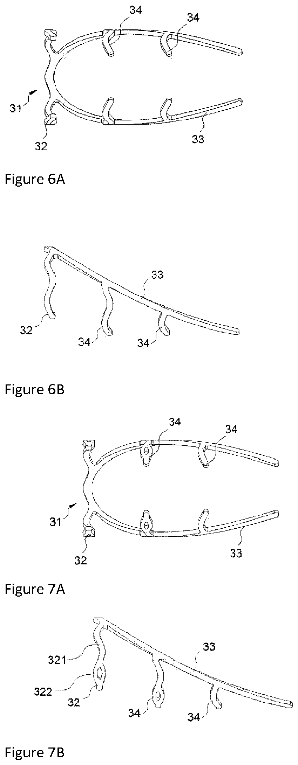

FIGS. 6A and 6B are respectively a front view and a side view of the rib component structure according to another embodiment of the present application. In this embodiment, the circumferential rib 32 of the rib component 31, and the auxiliary circumferential ribs 34 may be serpentine or circumferentially S-shaped or other special-shaped ribs to further enhance the circumferential supporting of the circumferential rib 32 and the auxiliary circumferential ribs 34 for the transitional connection segment.

FIGS. 7A and 7B are respectively a front view and a side view of the rib component structure according to still another embodiment of the present application. In this embodiment, the circumferential rib 32 of the rib component 31, and the auxiliary circumferential ribs 34 each include a basic segment and a reinforcement segment, for example, the basic segment 321 and the reinforcement segment 322 located on the circumferential rib 32 as shown in FIG. 7B, and the reinforcement segment 322 is provided with an eyelet to form branches that are wider or have a larger surface area than the basic segment 321, so as to further enhance the circumferential support of the circumferential rib 32 and the auxiliary circumferential ribs 34 to the transitional connection segment.

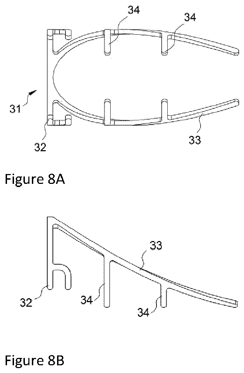

FIGS. 8A and 8B are respectively a front view and a side view of the rib component structure according to yet another embodiment of the present application. In this embodiment, the circumferential rib 32 of the rib component 31, and the auxiliary circumferential ribs 34 each include a basic segment and a reinforcement segment, there are branches on the reinforcement segment, and the branches and the main body are assembled into a whole by means of welding, soldering, bonding, or other connection methods to meet different performance requirements.

The exemplary embodiments in this description provide methods by way of example only, and examples of one method are not intended to limit examples of another method. A device/method illustrated in a figure may be added to or exchanged with devices/methods in other figures. Furthermore, specific digital data values (for example, specific numbers, quantities, categories, etc.) or other specific information are used merely to describe the exemplary embodiments and are not intended to limit the exemplary embodiments with such specific information.

## Claims

1. A guide extension catheter, comprising:
a guide tube, which comprises a proximal port and a distal port and comprises an inner layer, an intermediate layer, and an outer layer;
a guide shaft, which is disposed on the side of proximal port and configured to guide the guide tube into a target position;
a transitional connection segment, which is configured to connect the guide tube and the guide shaft, the transitional connection segment being of a tubular structure that axially extends from the proximal port and comprises an inclined opening,
wherein the transitional connection segment comprises an inner-layer extension segment, an outer-layer extension segment, and a rib component; the rib component is sized and shaped to adapt to the transitional connection segment; and the inner-layer extension segment is a part of the inner layer, and the outer-layer extension segment is a part of the outer layer;
wherein the rib component and the intermediate layer are coaxial with but not connected to each other, and are spaced apart by a first spacing of 0.1 mm to 10 mm; and the rib component and the guide shaft are spaced apart by a distance but not connected to each other, and are spaced apart by a third spacing of 0.1 mm to 2 mm; and
wherein the intermediate layer, the rib component, the inner layer and the inner-layer extension segment are wrapped by the outer layer and the outer-layer extension segment and are then hot-melt welded together.

2. The guide extension catheter according to claim 1, wherein the rib component comprises a circumferential rib and an oblique rib, the oblique rib having a profile corresponding to the shape of the inclined opening; and the circumferential rib and the oblique rib form an overlapping connection segment at the proximal port, such that the circumferential rib and the oblique rib form an integral structure as a whole.

3. The guide extension catheter according to claim 2, wherein the circumferential rib is C-shaped and comprises two arc-shaped ribs circumferentially extending from two ends of the overlapping connection segment.

4. The guide extension catheter according to claim 2, wherein the circumferential rib is a circumferential closed ring that passes the overlapping connection segment.

5. The guide extension catheter according to claim 2, wherein the oblique rib is C-shaped and comprises a left rib and a right rib which are sized and positioned corresponding to the inclined opening, and multiple sets of auxiliary circumferential ribs are distributed within a length range of the left rib and the right rib to support the circumferential stiffness of the transitional connection segment.

6. The guide extension catheter according to claim 5, wherein tail portions of the auxiliary circumferential ribs do not overlap each other, and are spaced apart by a distance.

7. The guide extension catheter according to claim 5, wherein the circumferential rib or the auxiliary circumferential rib is circumferentially S-shaped.

8. The guide extension catheter according to claim 5, wherein the circumferential rib or the auxiliary circumferential rib comprises a basic segment and a reinforcement segment, the reinforcement segment having a larger width or surface area than the basic segment.

9. The guide extension catheter according to claim 8, wherein the reinforcement segment comprises a branch or an eyelet.

10. The guide extension catheter according to claim 1, wherein the rib component is made of a nickel-titanium alloy, a cobalt alloy, a titanium alloy, a platinum-iridium alloy, stainless steel, or a carbon fiber material.

11. The guide extension catheter according to claim 1, wherein the outer layer is made of nylon, polyurethane or a thermoplastic elastomer, and the inner layer is made of a polymer; and the intermediate layer is a metal mesh layer.

12. The guide extension catheter according to claim 10, wherein the rib component is a round wire or a square bar.

13. The guide extension catheter according to claim 1, wherein, the rib component comprises a C-shaped circumferential rib and a C-shaped oblique rib, the oblique rib having a profile corresponding to the shape of the inclined opening; the circumferential rib and the oblique rib form an overlapping connection segment at the proximal port, such that the circumferential rib and the oblique rib form an integral structure as a whole; and the oblique rib comprises a left rib and a right rib which are sized and positioned corresponding to the inclined opening, and multiple sets of auxiliary circumferential ribs are distributed within the length range of the left rib and the right rib to support the circumferential stiffness of the transitional connection segment.
